# EUROPEAN PATENT SPECIFICATION

(11) **EP 4 073 738 B1**
(45) Date of publication and mention of the grant of the patent: **01.04.2026**
(21) Application number: 20899693.4
(22) Date of filing: 09.10.2020
(51) Int. Cl.: G16H 10/60, G16H 10/40, G16H 40/20, G16H 20/10, G16H 50/20, G16H 50/70, G06N 3/02, G06N 20/00, G06Q 50/00, G06Q 30/06

(54) **METHOD OF ANALYZING DIAGNOSTIC TEST ORDERS**
VERFAHREN ZUR ANALYSE DIAGNOSTISCHER TESTAUFTRÄGE
PROCÉDÉ D'ANALYSE DE COMMANDES DE TESTS DIAGNOSTIQUES

(30) Priority: 10.12.2019 US 201962946139 P
(43) Date of publication of application: 19.10.2022
(73) Proprietor: Siemens Healthcare Diagnostics Inc., Tarrytown, NY 10591 (US)
(72) Inventor: HEYDLAUF, Michael, Cary, North Carolina 27519 (US)
(74) Representative: Schweitzer, Klaus
(86) International application number: PCT/US2020/070636
(87) International publication number: WO 2021/119643

(56) References cited:
- WO-A2-2007/103303
- US-A1- 2003 092 980
- US-A1- 2015 046 181
- US-A1- 2015 356 252
- US-A1- 2018 004 901
- US-A1- 2018 098 736
- US-A1- 2018 268 934
- US-A1- 2019 355 481

## Description

### FIELD

Embodiments of the present disclosure relate to apparatus, systems, and methods of analyzing diagnostic test orders.

### BACKGROUND

Medical providers, such as physicians, may order diagnostic tests, such as panels of diagnostic tests, on specimens taken from patients seeking and/or undergoing medical treatment. Medical providers may choose from numerous different tests to help diagnose such patients.

Document US 2019/355481 A1 discloses real-time computer systems and software products connected to medical diagnosis, procedure coding, insurance, and billing systems, and programmed to detect and pre-empt abuse, fraud, and excessive profits by medical insiders and institutions.

Document US 2015/046181 A1 discloses a laboratory virtual healthcare assistant configured to facilitate communication between care providers and a lab system, which may notify of any duplicate, missing, or mistakenly ordered lab tests.

### SUMMARY

In a first aspect, a method of analyzing diagnostic test orders is provided. The method includes analyzing diagnostic test orders from a plurality of medical providers; identifying a peer group of medical providers having one or more like characteristics from the plurality of medical providers; and determining whether at least one diagnostic test order includes an outlier test.

In another aspect, a method of operating a diagnostic test system is disclosed. The method includes receiving one or more diagnostic test orders from a first medical provider; establishing a peer group based on one or more like characteristics of the first medical provider and one or more second medical providers; determining historical diagnostic test order data for the peer group; identifying one or more differences between one or more diagnostic tests ordered by the first medical provider and historical diagnostic tests ordered by the peer group; and determining whether the one or more diagnostic tests include an outlier test.

In another aspect, a diagnostic test system is disclosed. The system includes a processor and memory, the memory including instructions executable on the processor to: analyze one or more diagnostic test orders from a plurality of medical providers, identify a peer group of medical providers having one or more like characteristics from the plurality of medical providers, and determine whether at least one diagnostic test order includes an outlier test.

### BRIEF DESCRIPTION OF THE DRAWINGS

The drawings, described below, are for illustrative purposes and are not necessarily drawn to scale. Accordingly, the drawings and descriptions are to be regarded as illustrative in nature, and not as restrictive. The drawings are not intended to limit the scope of the disclosure in any way.
FIG. 1 illustrates a schematic diagram of a diagnostic test system according to one or more embodiments.
FIG. 2 illustrates a flowchart describing a method of analyzing diagnostic test orders according to one or more embodiments.
FIG. 3 illustrates a flowchart describing a method of using a diagnostic test system according to one or more embodiments.
FIG. 4 illustrates a flow chart describing a method of analyzing diagnostic test orders according to one or more embodiments.
FIG. 5 illustrates a flowchart describing a method of using a diagnostic test system according to one or more embodiments.

### DETAILED DESCRIPTION

As a part of a patient evaluation, medical providers may order one or more diagnostic tests on specimens (e.g., blood, urine, feces, sputum, cerebral-spinal fluid, etc.) taken from the patient. For example, medical providers with certain characteristics may order certain diagnostic tests or panels of diagnostic tests over time. The diagnostic tests described herein may refer to panels of diagnostic tests. The characteristics of the medical providers may include one or more medical specialties of the medical providers, patient and/or medical provider demographics, population health, cultural norms, patient symptoms, and other identifying characteristics. Population health may include diseases that the patient population may be subject to given demographics, location, and time of year. Over time, the diagnostic tests ordered by a first medical provider may differ from diagnostic tests ordered by his or her peers. The diagnostic tests ordered by his or her peers may include a plurality of tests ordered over a period of time and may include a statistically-relevant number of tests in some cases. The differences in one or more diagnostic tests ordered by the first medical provider and a plurality of diagnostic tests ordered by second medical providers (e.g., peers of the first medical provider) may be referred to as an outlier test(s) and/or a test anomaly(ies).

The first medical provider and some of his or her peers may be unaware of an outlier test(s). Thus, some medical providers with like characteristics (e.g., specialties and other like identifying characteristics) may be ordering different diagnostic tests than their peers without knowledge that they are ordering the different diagnostic tests. In some situations, the second medical providers may be ordering more diagnostic tests than the first medical provider. In these situations, the additional diagnostic test(s) ordered by the second medical providers may be an outlier test(s). In other situations, the first medical provider may be ordering more diagnostic tests than the second medical providers. In these situations, the additional test(s) ordered by the first medical provider may be considered an outlier test(s). The outlier test(s) may be identified and reviewed by one or more of the medical providers. For example, in some situations, a medical provider may commence ordering the outlier test(s) and in other situations, a medical provider may stop ordering the outlier test(s). In some instances, a first medical provider may have ordered an outlier test and the other medical providers may adopt ordering that outlier test, as feedback on the reasons for the test may be provided from the first medical provider.

Methods, apparatus, and systems disclosed herein identify an outlier test(s). In some method embodiments, one or more characteristics of a first medical provider may be identified. Second medical providers having like characteristics as the first medical provider may be identified. These second medical providers may be peers with the first medical provider in that they have like immutable characteristics. Diagnostic tests ordered by the first medical provider and the second medical providers may be identified. Differences between diagnostic tests ordered by the first medical provider and diagnostic tests ordered by the second medical providers may be identified. These differences may be identified as an outlier test(s). By using the term "outlier test" it is not meant to indicate that the test is improper, as further discussion/analysis/reasoning provided by the medical provider(s) may deem that the outlier test(s) is not only desirable, but adds additional information for determining or excluding certain diseases or ailments.

In some cases, the outlier test may indicate a deprecated test; when, for example, a new higher-sensitivity test for the same marker becomes available. In other cases, the outlier test may represent a new diagnostic option not previously known to a medical provider. In some embodiments, a signal may be generated notifying the medical providers of an outlier test. The signal may result in a report or other notification being generated. The report or other notification may include statistics concerning the prevalence of the diagnostic tests ordered by the first medical provider and may include statistics concerning the prevalence of any outlier test(s). As should be recognized, more than one outlier test may be identified.

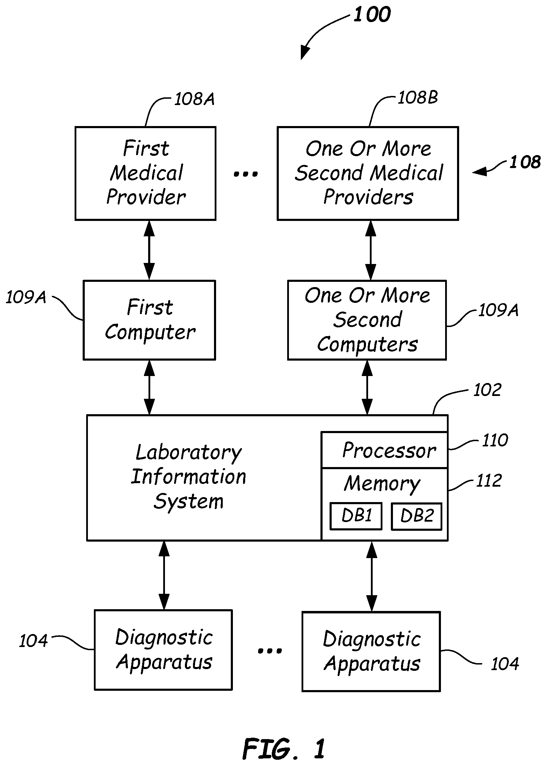

These and other methods, apparatus, and systems are described in further detail herein with reference to FIGs. 1-5.

Reference is now made to FIG. 1, which illustrates a schematic diagram of a diagnostic test system 100, which may be a component of a laboratory automation system. The diagnostic test system 100 may include a laboratory interface system (LIS) 102 that may receive diagnostic test orders and communicate with a diagnostic apparatus 104, which may be any suitable analyzer (e.g., clinical diagnostic analyzer, immune-assay instrument, or the like) to complete the diagnostic test orders. For example, the diagnostic apparatus 104 may perform testing on one or more specimens per each diagnostic order. In some embodiments, the LIS 102 may communicate with a plurality of diagnostic apparatus. The LIS 102 may receive diagnostic test orders from a plurality of sources, such as a plurality of medical providers 108 (e.g., physicians). In the embodiment depicted in FIG. 1, the LIS 102 may receive diagnostic test orders from at least a first medical provider 108A and also from one or more second medical providers 108B. For example, a medical provider may instruct personnel (e.g., phlebotomist) to take a specimen(s) (e.g., liquid or other specimen(s)) from a patient and order certain diagnostic tests to be performed on the specimen(s).

The instructions may be transmitted from the first medical provider 108A to the LIS 102 via a first computer 109A, such as a workstation or other device. Information, such as reports and the like described herein, may be transmitted from the LIS 102 to the first computer 109A. Instructions also may be transmitted from the one or more second medical providers 108B to the LIS 102 via one or more second computers 109B, such as a workstation or other device. Information, such as reports and the like described herein, may be transmitted from the LIS 102 to the one or more second computers 109B.

As described above, the LIS 102 may receive diagnostic test orders from medical providers 108, including the first medical provider 108A and the one or more second medical providers 108B. The one or more second medical providers 108B may include a statically-relevant number of medical providers. The LIS 102 or a computer in communication with the LIS 102 may track the patient samples and instruct the diagnostic apparatus 104 to perform the one or more diagnostic tests. The LIS 102 may receive diagnostic test results from the diagnostic apparatus 104 and transmit the diagnostic test results to the requesting medical provider.

The diagnostic test system 100 may include a processor 110 and memory 112. In the embodiment depicted in FIG. 1, the processor 110 and/or the memory 112 may be included in or in communication with the LIS 102. The memory 112 may include one or more databases that store data used in the processing described herein. For example, a first database DB1 may store diagnostic test orders ordered by the medical providers 108. A second database DB2 may store characteristics of the medical providers 108 as described herein. Optionally a single database may store diagnostic test orders and characteristics of the medical providers 108.

The memory 112 may also store program instructions that when executed by the processor 110 perform tasks described herein. For example, the processor 110 may compare diagnostic test order data stored in the memory 112 and identify one or more outlier tests and notify the first medical provider 108A of the one or more outlier tests. The one or more second medical providers 108B may also be informed of the one or more outlier tests as described herein. The processor 110 may also identify one or more like characteristics of the medical providers 108.

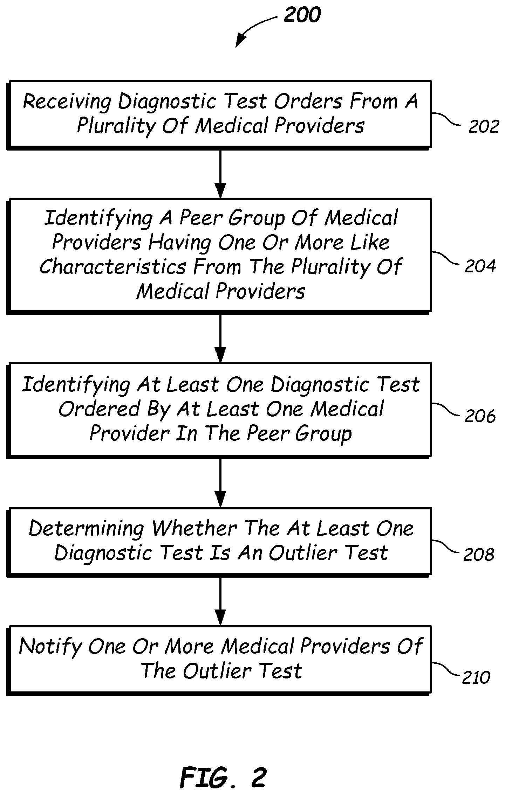

Additional reference is made to FIG. 2, which illustrates a flowchart describing a method 200 of analyzing diagnostic test orders. The method 200 may be performed, at least in part, by the processor 110 (FIG. 1) executing programmed computer instructions stored in the memory 112 (FIG. 1). The method 200 includes, in block 202, receiving diagnostic test orders from a plurality of medical providers. The plurality of medical provides may include the medical providers 108 (FIG. 1), including the first medical provider 108A (FIG. 1) and the one or more second medical providers 108B (FIG. 1). The diagnostic test orders may include diagnostic tests that may be performed by the diagnostic apparatus 104 (FIG. 1).

The method 200 includes, in block 204, identifying a peer group of medical providers having one or more like characteristics from the plurality of medical providers 108. The one or more like characteristics may include one or more medical provider specialties and/or disciplines, patient demographics (e.g., age, sex, race, nationality, etc. of the patients), population health (e.g., whether a disease is prevalent in the population), medical provider demographics, symptoms being treated by the plurality of medical providers, reasons patients seek treatment from the plurality of medical providers, and one or more regional and/or cultural norms of patients and/or the plurality of medical providers. The peer group may be based on other like characteristics (e.g., identifying characteristics).

The identification of a peer group may be performed by the processor 110 (FIG. 1), wherein instructions to perform the identification may be stored in the memory 112 (FIG. 1). The processor 110 may run one or more support vector machines, one or more artificial neural networks, and/or artificial intelligence to identify the peer group. Other algorithms may be run to identify the peer group. A support vector machine (SVM) may include a discriminative classifier defined by a separating hyperplane. The SVM may be an algorithm that is provided with labeled training data (characteristics), the algorithm may output an optimal hyperplane, which categorizes new characteristics. In other embodiments, the characteristics of the medical providers may be stored in a database in memory 112, wherein the processor 110 analyzes the database to identify medical providers having like characteristics.

The method 200 includes, in block 206, identifying at least one diagnostic test ordered by at least one medical provider in the peer group. For example, at least one diagnostic test ordered by a first medical provider 108A (FIG. 1) may be analyzed in the LIS 102. In some embodiments, the processor 110, running programmed computer instructions stored in the memory 112, may run one or more algorithms that identify the at least one diagnostic test.

The method 200 includes in block 208, determining whether the at least one diagnostic test is an outlier test. In some embodiments, the method may analyze historic diagnostic tests ordered by the peer group and compare those historic diagnostic tests to the one or more diagnostic tests ordered by at least one medical provider. In some embodiments, anomaly and/or outlier techniques may be used to determine if any at least one diagnostic test is an outlier test (e.g., an anomaly). Thus, the processor 110, for example, may analyze all the diagnostic tests ordered by the peer group to determine if any one of the one or more of the diagnostic tests is an outlier test. The outlier determination may be performed by the processor 110 (FIG. 1), wherein instructions to perform the outlier determination may be stored in the memory 112 (FIG. 1). The processor 110 may run one or more support vector machines, one or more artificial neural networks, and/or artificial intelligence to make the outlier determination. In other embodiments, the diagnostic test orders may be stored in a database, wherein the processor 110 analyzes the database to identify any outlier test(s).

If the one or more tests are determined to include an outlier test, one or more medical providers may be notified in block 210. In some embodiments, the first medical provider 108A is notified. In some embodiments, medical providers, such as medical providers in the peer group, may also be notified of the outlier test. In some embodiments, an outlier test is a diagnostic test that is ordered by less than a predetermined number or less than a predetermined percentage of the medical providers in the peer group. In some embodiments, an outlier test includes a diagnostic test ordered by the one or more second medical providers 108B (FIG. 1), but not by a first medical provider 108A (FIG. 1). In some embodiments, an outlier test includes a diagnostic test ordered by the first medical provider 108A, but not by the one or more second medical providers 108B. In some embodiments, an outlier test includes a diagnostic test ordered by a first group of medical providers, but not by a second group of medical providers. As described above, the outlier tests may be panels of tests.

In some embodiments, an outlier test is a diagnostic test that is ordered by less than a predetermined percentage of medical providers in a peer group. In some embodiments, a diagnostic test that is ordered by the first medical provider 108A, but is only ordered by 50% or fewer of the one or more second medical providers 108B is considered to be an outlier test. In another example, a diagnostic test that is ordered by greater a predetermined percentage of the one or more second medical providers 108B, but is not ordered by the first medical provider 108A is an outlier test.

In some embodiments, identifying the one or more characteristics of the plurality of medical providers and/or determining whether at least one diagnostic test is an outlier test comprises using artificial intelligence. In some embodiments, identifying the one or characteristics of the plurality of medical providers and/or determining whether at least one diagnostic test is an outlier test comprises using k-means clustering. The number of clusters may equal the number of characteristics being identified or the number of diagnostic tests being analyzed to determine whether an outlier test exists.

Additional reference is made to **Table 1,** which illustrates scenarios of outlier tests. **Table 1** illustrates a first scenario wherein a first group of medical providers historically orders diagnostic tests A-D. The first medical provider 108A (FIG. 1) has ordered diagnostic tests A-F or historically orders diagnostic tests A-F. The first group of medical providers illustrated in **Table 1** may include the one or more second medical providers 108B (FIG. 1). Both the first group of medical providers and first medical provider may be in the same peer group. In this scenario, diagnostic tests E and F may be outlier tests because the medical providers in the first group have not historically ordered diagnostic tests E and F. For example, diagnostic tests E and F may be ordered by less than a predetermined percentage of the first group of medical providers. In some embodiments, less than 50% of the medical providers in the first group may order diagnostic tests E and F. In other embodiments, less than 30%, or less than 20%, or less than 10% of the medical providers in the first group may order diagnostic tests E and F.

Optionally, diagnostic tests E and F may be ordered less than a predetermined percentage of the time by the first group of medical providers for a suspected disease or ailment or in combination with a panel of other diagnostic tests. In other embodiments, the panel of diagnostic tests A-F may be less than a predetermined percentage of overall diagnostic tests ordered by the first group of medical providers.

**Table 1 - Test Scenarios**

| Scenario | Diagnostic tests ordered by first group of X medical providers | Diagnostic tests ordered by second group of Y medical providers | Diagnostic tests ordered by first medical provider | Outlier tests |
|---|---|---|---|---|
| 1 | A-D | | A-F | E and F |
| 2 | A-D | B-E | A-F | F |
| 3 | A-D | B-E | A-C | D and E |

In the first scenario, the processor 110 (FIG. 1) may generate a signal indicating that diagnostic tests E and F are outlier tests. The processor 110 may notify the first medical provider 108A and/or the one or more second medical providers 108B of the outlier tests. In some embodiments, the outlier tests may be discontinued by the first medical provider 108A or optionally adopted by the one or more second medical providers 108B, such as after due consideration of the value of the outlier tests.

In some embodiments, the processor 110 may generate a report providing statistical analysis of one or more diagnostic tests ordered by the first medical provider 108A and/or the one or more second medical providers 108B. The statistical analysis may indicate the number of the one or more second medical providers 108B, the percentage of the one or more second medical providers 108B that order certain diagnostic tests, the characteristics that constitute the peer group, and/or other information.

**Table 1** also illustrates a second scenario wherein X number of medical providers (e.g., the first group) in a peer group historically order diagnostic tests A-D and Y number of medical providers (e.g., a second group) in the peer group historically order diagnostic tests B-E. The first group of medical providers combined with the second group of medical providers may constitute the one or more second medical providers 108B (FIG. 1). The first medical provider 108A (FIG. 1), who is in the peer group, has ordered or historically orders diagnostic tests A-F. In this scenario, diagnostic test F is an outlier test because neither the medical providers in the first group nor the medical providers in the second group historically order diagnostic test F. In some embodiments, the panel of diagnostic tests A-F is considered to be an outlier test. In some embodiments, the panel of diagnostic tests A-F may be ordered by less than a predetermined percentage (e.g. less than 50%) of the medical providers in the peer group, which may indicate that the panel of diagnostic tests A-F is an outlier panel.

In some embodiments, the first group of medical providers may historically order diagnostic tests A-D and the second group of medical providers may historically order diagnostic tests B-E. Accordingly, diagnostic tests A and E may not be outlier tests. If, on the other hand, the first group of medical providers rarely orders tests A-D or the second group of medical providers rarely orders tests B-E, then one of the diagnostic tests A or E may be an outlier test in addition to diagnostic test F. For example, if the second group of medical providers rarely orders diagnostic tests B-E, then diagnostic test E and diagnostic test F are considered to be outlier tests. In some embodiments, diagnostic tests B-E may be ordered by 50% or less, or 20% or less, or less than 10% of the second group of medical providers, which may trigger the diagnostic tests E and F to be considered to be outlier tests. The processor 110 (FIG. 1) may generate a signal notifying one or more of the medical providers of the outlier test as described above.

In a third scenario, the first group of medical providers historically order diagnostic tests A-D, the second group of medical providers historically order diagnostic tests B-E, and the first medical provider 108A (FIG. 1) has ordered or historically orders diagnostic tests A-C. In this scenario, diagnostic tests D and E are considered to be outlier tests. For example, the first group of medical providers historically order diagnostic test D and the second group of medical providers historically order diagnostic test E. If the second group rarely orders diagnostic test E, then diagnostic test E may be an outlier test. For example, if less than 50%, or 20% or less, or 10% or less of the second group orders diagnostic test E, then diagnostic test E is considered to be an outlier test.

As described above, the processor 110 (FIG. 1) may notify one or more of the medical providers of the outlier tests. For example, the processor 110 may notify the first medical provider that his or her peers are ordering diagnostic tests D and E.

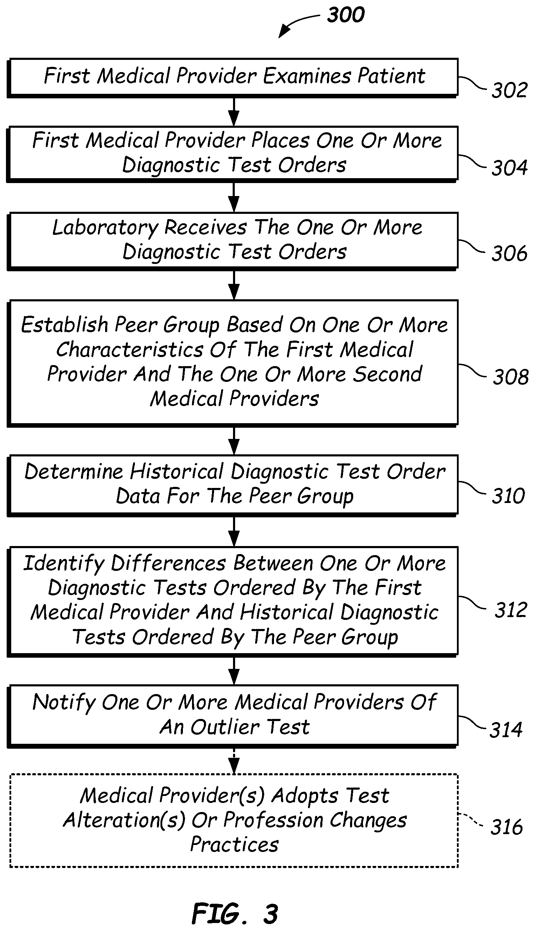

Additional reference is made to FIG. 3, which is a flowchart describing an embodiment of a method 300 of using the diagnostic test system 100 (FIG. 1). The method may commence at block 302 with the first medical provider 108A (FIG. 1) examining a patient. In block 304, the first medical provider 108A may place one or more diagnostic test orders in response to analyzing the patient. For example, the first medical provider 108A may analyze symptoms of the patient and place the one or more diagnostic test orders to diagnose one or more diseases or ailments of the patient. In block 306, the laboratory receives the one or more diagnostic test orders from the first medical provider 108A. For example, the first medical provider 108A may input the diagnostic test orders into a computer in a medical office and the diagnostic test orders may be transmitted to and received in the laboratory via the LIS 102 (FIG. 1). In some embodiments, the computer may be part of the LIS 102.

In block 308 a peer group of medical providers may be established. For example, software, including programmed computer instructions stored in the memory 112 (FIG. 1), may execute on the processor 110 (FIG. 1) to establish the peer group. The peer group may be based on one or more characteristics of the first medical provider 108A and like characteristics of the one or more second medical providers 108B. The one or more characteristics may include, for example, medical professional specialty and/or discipline, patient demographics (e.g., age, sex, race, nationality, etc. of the patients), symptoms of patient(s) being treated, reasons for patient(s) treatment, population health, and regional and/or cultural norms. Other characteristics may be used to establish the peer group.

In block 310, historical diagnostic test order data is determined for the peer group. For example, computer instructions stored in the memory 112 (FIG. 1) may run on the processor 110 (FIG. 1) to determine diagnostic tests historically ordered by the peer group. In some embodiments, artificial intelligence may be used, such as by the processor 110, to create the peer group and/or determine the historic diagnostic test orders. In some embodiment, analysis of diagnostic tests historically ordered may be accomplished by k-means clustering. In some embodiments, the number of clusters may be the number of different diagnostic tests historically ordered by the peer group.

Differences between diagnostic tests ordered, such as by the first medical provider 108A and historic diagnostic tests ordered by the peer group, may be identified in block 312. For example, methods to identify anomaly and/or outlier tests may be used to determine if any diagnostic tests in the peer group are outlier tests. In some embodiments, one or more diagnostic tests ordered by the peer group including the one or more second medical providers 108B may be analyzed to determine if one or more tests ordered by the first medical provider 108A are outlier tests. Thus, the processor 110, for example, may analyze all the diagnostic tests ordered by the peer group including the first medical provider 108A and the one or more second medical providers to determine if one or more of the diagnostic tests include any outlier tests. In similar embodiments, the processor 110 may, for example, determine if one or more diagnostic tests ordered by the first medical provider 108A comprise an outlier test relative to one or more diagnostic tests historically ordered by the one or more second medical providers 108B in the peer group.

In block 314, one or more of the medical providers may be notified of an outlier test and/or an anomaly. For example, the processor 110 may send a notification to the first medical provider 108A indicating that the first medical provider 108A is ordering or has ordered one or more diagnostic tests not ordered by his or her peers or that the first medical provider 108A is not ordering or has not ordered one or more diagnostic tests ordered by his or her peers. In some embodiments, the processor 110 may send a notification to the first medical provider 108A indicating that the first medical provider is ordering or has ordered one or more panel of diagnostic tests not ordered by his or her peers or that the first medical provider 108A is not ordering or has not ordered one or more panels of diagnostic tests ordered by his or her peers. For example, the notification may be sent if the outlier tests include diagnostic tests ordered or not ordered for a specific type or characteristic of patient. In some embodiments, the outlier test is included in a panel of diagnostic tests.

In block 316, medical provider(s) may revise their diagnostic testing protocols in response to the outlier test(s). For example, the first medical provider 108A may disregard one or more outlier tests, such as diagnostic test F in the first scenario of **Table 1.** In other embodiments, the one or more second medical providers 108B may adopt the one or more outlier test(s) into their diagnostic testing protocol.

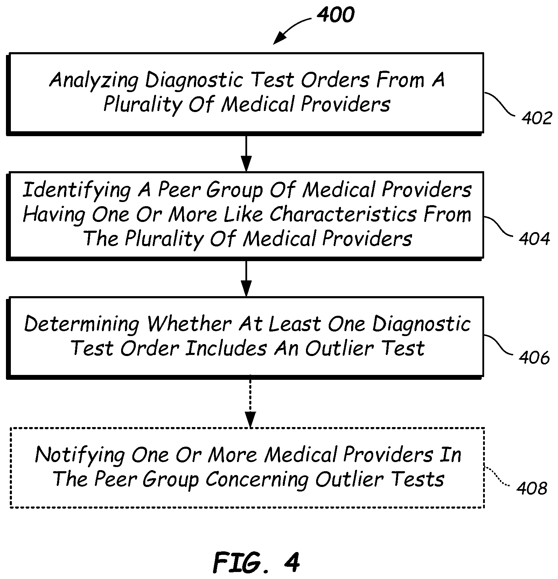

Reference is now made to FIG. 4, which illustrates a method 400 of analyzing diagnostic test orders. The method 400 includes, in block 402, analyzing diagnostic test orders from a plurality of medical providers (e.g., medical providers 108). The method 400 includes, in block 404, identifying a peer group of medical providers having one or more like characteristics from the plurality of medical providers. The method 400 includes, in block 406, determining whether at least one diagnostic test order includes an outlier test. In block 408, the method 400 may optionally include notifying one or more medical providers in the peer group concerning the outlier tests.

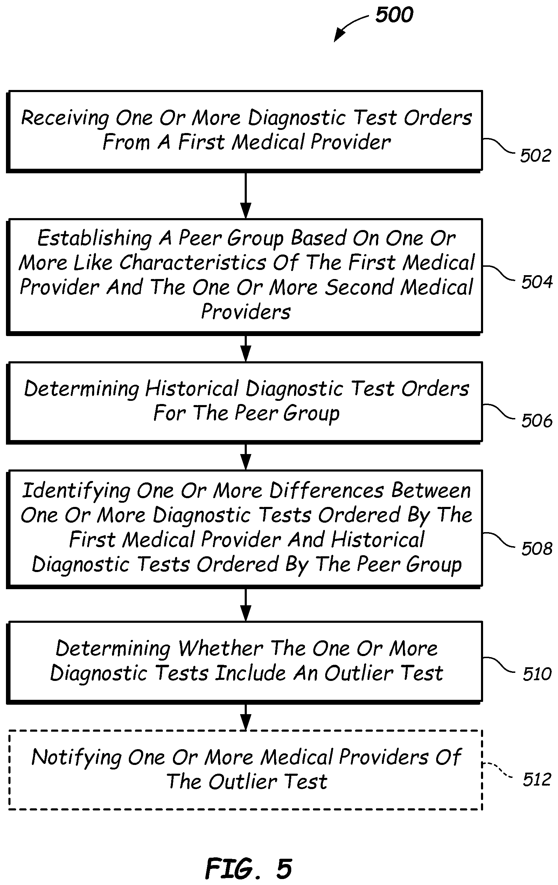

Reference is now made to FIG. 5, which illustrates a flowchart describing a method 500 of operating a diagnostic test system (e.g., diagnostic test system 100). The method 500 includes, in block 502, receiving one or more diagnostic test orders from a first medical provider (e.g., first medical provider 108A). The method 500 includes, in block 504, establishing a peer group based on one or more like characteristics of the first medical provider and the one or more second medical providers. The method 500 includes, in block 506, determining historical diagnostic test orders for the peer group. The method 500 includes, in block 508, identifying one or more differences between one or more diagnostic tests ordered by the first medical provider and historical diagnostic tests ordered by the peer group. The method 500 includes, in block 510, determining whether the one or more diagnostic tests include an outlier test. The method 500 includes, in block 512, notifying one or more medical providers of the outlier test. In particular, the first medical provider is notified so that he or she adjusts his or her test ordering to accept or reject the outlier test. In some embodiments, the medical provider may convince the peer group that such a test order is warranted.

## Claims

1. A computer-implemented method of operating a diagnostic test system, comprising receiving one or more diagnostic test orders from a first medical provider;
establishing a peer group based on one or more like characteristics of the first medical provider and one or more second medical providers;
determining historical diagnostic test orders for the peer group;
identifying one or more differences between one or more diagnostic tests ordered by the first medical provider and historical diagnostic tests ordered by the peer group; and
determining whether the one or more diagnostic tests include an outlier test, notifying one or more medical providers of the outlier test comprising notifying the first medical provider; and
adjusting, by the first provider, the test ordering by accepting or rejecting the outlier test.

2. The method of claim 1, wherein the outlier test includes a test ordered by the one or more second medical providers, but not by the first medical provider, or wherein the outlier test includes a test ordered by the first medical provider, but not by the one or more second medical providers.

3. The method of claim 1, wherein the one or more like characteristics include one or more medical specialties.

4. The method of claim 1, wherein the one or more like characteristics include one or more demographics of the medical providers.

5. The method of claim 1, wherein the one or more like characteristics include one or more demographics of patients of the medical providers, or
wherein the one or more like characteristics include one or more cultural norms of the medical providers, or
wherein the one or more like characteristics include one or more cultural norms of patients of the medical providers, or
wherein the one or more like characteristics include one or more symptoms of patients.

6. The method of claim 1, wherein the outlier test is a diagnostic test included in a panel of diagnostic tests ordered by a first group of medical providers in the peer group, but not by a second group of medical providers in the peer group.

7. The method of claim 1, wherein determining whether the at least one diagnostic test order includes an outlier test comprises using artificial intelligence.

8. The method of claim 1, wherein determining whether the at least one diagnostic test order includes an outlier test includes using k-means clustering.

9. The method of claim 1, wherein determining whether the at least one diagnostic test order includes an outlier test comprises using a support vector machine.

10. The method of claim 1, wherein determining whether the at least one diagnostic test order includes an outlier test comprises using one or more artificial neural networks.

## Patentansprüche

1. Computerimplementiertes Verfahren zum Betreiben eines diagnostischen Testsystems, umfassend
Empfangen eines diagnostischen Testauftrags oder mehrerer diagnostischer Testaufträge von einem ersten medizinischen Anbieter;
Einrichten einer Peer-Gruppe basierend auf einem oder mehreren ähnlichen Merkmal (en) des ersten medizinischen Anbieters und eines zweiten medizinischen Anbieters oder mehrerer zweiter medizinischer Anbieter;
Bestimmen historischer diagnostischer Testaufträge für die Peer-Gruppe;
Identifizieren eines Unterschieds oder mehrerer Unterschiede zwischen einem oder mehreren diagnostischen Test(s), der/die durch den ersten medizinischen Anbieter beauftragt ist/sind, und historischen diagnostischen Tests, die durch die Peer-Gruppe beauftragt sind; und
Bestimmen, ob der eine oder die mehreren diagnostische(n) Test(s) einen Ausreißertest beinhaltet/beinhalten, Benachrichtigen eines medizinischen Anbieters oder mehrerer medizinischer Anbieter über den Ausreißertest, umfassend ein Benachrichtigen des ersten medizinischen Anbieters; und
Anpassen, durch den ersten Anbieter, der Testauftragserteilung durch Annehmen oder Ablehnen des Ausreißertests.

2. Verfahren nach Anspruch 1, wobei der Ausreißertest einen Test beinhaltet, der durch den einen oder die mehreren zweiten medizinischen Anbieter, aber nicht durch den ersten medizinischen Anbieter beauftragt ist, oder wobei der Ausreißertest einen Test beinhaltet, der durch den ersten medizinischen Anbieter, aber nicht durch den einen oder die mehreren zweiten medizinischen Anbieter beauftragt ist.

3. Verfahren nach Anspruch 1, wobei das eine oder die mehreren ähnliche(n) Merkmal(e) ein oder mehrere medizinische(s) Fachgebiet(e) beinhaltet/beinhalten.

4. Verfahren nach Anspruch 1, wobei das eine oder die mehreren ähnliche(n) Merkmal(e) eine oder mehrere Demografie(n) der medizinischen Anbieter beinhaltet/beinhalten.

5. Verfahren nach Anspruch 1, wobei das eine oder die mehreren ähnliche(n) Merkmal(e) eine oder mehrere Demografie(n) von Patienten der medizinischen Anbieter beinhaltet/beinhalten oder wobei das eine oder die mehreren ähnliche(n) Merkmal(e) eine oder mehrere kulturelle Norm(en) der medizinischen Anbieter beinhaltet/beinhalten oder
wobei das eine oder die mehreren ähnliche(n) Merkmal(e) eine oder mehrere kulturelle Norm(en) von Patienten der medizinischen Anbieter beinhaltet/beinhalten oder
wobei das eine oder die mehreren ähnliche(n) Merkmal(e) ein oder mehrere Symptom(e) von Patienten beinhaltet/beinhalten.

6. Verfahren nach Anspruch 1, wobei der Ausreißertest ein diagnostischer Test ist, der in einem Panel von diagnostischen Tests enthalten ist, die durch eine erste Gruppe von medizinischen Anbietern in der Peer-Gruppe, aber nicht durch eine zweite Gruppe von medizinischen Anbietern in der Peer-Gruppe beauftragt sind.

7. Verfahren nach Anspruch 1, wobei das Bestimmen, ob der mindestens eine diagnostische Testauftrag einen Ausreißertest beinhaltet, ein Verwenden von künstlicher Intelligenz umfasst.

8. Verfahren nach Anspruch 1, wobei das Bestimmen, ob der mindestens eine diagnostische Testauftrag einen Ausreißertest beinhaltet, ein Verwenden von k-Means-Clustering beinhaltet.

9. Verfahren nach Anspruch 1, wobei das Bestimmen, ob der mindestens eine diagnostische Testauftrag einen Ausreißertest beinhaltet, ein Verwenden einer Support-Vektor-Maschine umfasst.

10. Verfahren nach Anspruch 1, wobei das Bestimmen, ob der mindestens eine diagnostische Testauftrag einen Ausreißertest beinhaltet, ein Verwenden eines künstlichen neuronalen Netzwerks oder mehrerer künstlicher neuronaler Netzwerke umfasst.

## Revendications

1. Procédé, mis en œuvre par ordinateur, d'exploitation d'un système de test diagnostique comprenant
la réception d'une ou de plusieurs commandes de tests diagnostiques d'un premier prestataire médical ;
l'établissement d'un groupe de pairs sur la base d'une ou de plusieurs caractéristiques similaires du premier prestataire médical et d'un ou de plusieurs seconds prestataires médicaux ;
la détermination de demandes de tests diagnostiques historiques pour le groupe de pairs ;
l'identification d'une ou de plusieurs différences entre un ou plusieurs tests diagnostiques commandés par le premier prestataire médical et des tests diagnostiques historiques commandés par le groupe de pairs ; et
le fait de déterminer si le ou les tests diagnostiques comprennent un test atypique, une notification du test atypique à un ou plusieurs prestataires médicaux comprenant la notification du premier prestataire médical ; et
l'ajustement, par le premier fournisseur, de l'ordre des tests en acceptant ou en rejetant le test atypique.

2. Procédé selon la revendication 1, le test atypique comprenant un test commandé par le ou les seconds prestataires médicaux, mais pas par le premier prestataire médical, ou le test atypique comprenant un test commandé par le premier prestataire médical, mais pas par le ou les seconds prestataires médicaux.

3. Procédé selon la revendication 1, la ou les caractéristiques similaires comprenant une ou plusieurs spécialités médicales.

4. Procédé selon la revendication 1, la ou les caractéristiques similaires comprenant une ou plusieurs données démographiques des prestataires médicaux.

5. Procédé selon la revendication 1, la ou les caractéristiques similaires comprenant une ou plusieurs données démographiques de patients des prestataires médicaux, ou
la ou les caractéristiques similaires comprenant une ou plusieurs normes culturelles des prestataires médicaux, ou
la ou les caractéristiques similaires comprenant une ou plusieurs normes culturelles de patients des prestataires médicaux, ou
la ou les caractéristiques similaires comprenant un ou plusieurs symptômes de patients.

6. Procédé selon la revendication 1, le test atypique étant un test diagnostique inclus dans un panel de tests diagnostiques commandés par un premier groupe de prestataires médicaux dans le groupe de pairs, mais pas par un second groupe de prestataires médicaux dans le groupe de pairs.

7. Procédé selon la revendication 1, le fait de déterminer si la ou les commandes de test diagnostique comprennent un test atypique comprenant l'utilisation d'une intelligence artificielle.

8. Procédé selon la revendication 1, le fait de déterminer si la ou les commandes de test diagnostique comprennent un test atypique comprenant l'utilisation d'un regroupement à k centroïdes.

9. Procédé selon la revendication 1, le fait de déterminer si la ou les commandes de test diagnostique comprennent un test atypique comprenant l'utilisation d'une machine à vecteurs de support.

10. Procédé selon la revendication 1, le fait de déterminer si la ou les commandes de test diagnostique comprennent un test atypique comprenant l'utilisation d'un ou plusieurs réseaux de neurones artificiels.
